# DEMANDE DE BREVET EUROPEEN

(11) **EP 2 548 523 A1**
(43) Date de publication de la demande: **23.01.2013**
(21) Numéro de dépôt: 12177252.9
(22) Date de dépôt: 20.07.2012
(51) Int. Cl.: A61B 17/17

(54) **Instrumentation chirurgicale**

(30) Priorité: 26.07.2011 FR 1156826; 22.07.2011 US 201161510591 P
(71) Demandeur: Tornier, Inc., Edina, MN 55435 (US)
(72) Inventeur: Barouch Loïc, 38460 Carisieu (FR); Ferrand, Lucile, 38330 Montbonnot (FR); Hodorek, Brian C., Winona Lake, Indiana 46590 (US); Berger, Jean-Marie, Eden Prairie, MN 55344 (US); Gargac, Shawn, Fort Wayne, 46818 (US)
(74) Mandataire: Grand, Guillaume

(57) **Abrégé**

Afin d'améliorer le blocage en translation d'une tige (13) par rapport à un trou traversant (12) de réception de cette tige, délimité dans un ancillaire (10), l'invention propose de coincer la tige dans le trou traversant par une pièce de coincement (21) comprenant un corps allongé (22) qui, en service, s'étend en longueur de manière globalement parallèle à l'axe central (Z-Z) du trou traversant et qui est élastiquement déformable entre une configuration de repos et une configuration déformée, dans lesquelles un relief (25), qui fait transversalement saillie du corps en direction de l'axe central, est écarté vis-à-vis de l'axe central respectivement d'une distance radiale minimale strictement inférieure au rayon du trou traversant et d'une distance radiale minimale strictement supérieure au rayon du trou traversant, en passant par une configuration intermédiaire dans laquelle, alors que la tige est reçue dans le trou traversant, une surface d'appui (25A) délimitée par le relief est pressée contre la tige selon une direction transversale à l'axe central sous l'action du retour élastique du corps depuis sa configuration déformée vers sa configuration de repos.

## Description

La présente invention concerne une instrumentation chirurgicale, notamment une instrumentation permettant de mettre en place un clou d'ostéosynthèse.

Suite à la fracture d'un os long, tel que l'humérus, le fémur ou le tibia, on utilise fréquemment un clou d'ostéosynthèse, mis en place de manière étendue suivant sa longueur dans le canal médullaire de l'os. Ce clou permet de stabiliser les fragments d'os séparés par la fracture à traiter. Pour limiter autant que possible l'incision des chairs et autres parties molles autour du site de fracture osseuse, il est fréquent d'introduire le clou dans l'os, en l'enfilant par l'extrémité osseuse du canal médullaire, que l'on a préalablement ouverte uniquement dans le prolongement du canal. Dans ces conditions, on utilise ensuite un ancillaire de visée qui présente des trous traversants de guidage pour mettre en place, à plusieurs hauteurs du clou, des vis de fixation transversale entre le clou et l'os. Chacune de ces vis de fixation va être engagée de manière percutanée à l'intérieur d'un passage complémentaire délimité à travers le clou, le positionnement relatif adéquat entre le clou de guidage et le passage précité étant assuré par le fait que l'ancillaire de visée est lié mécaniquement, de manière prédéterminée, au clou logé dans le canal médullaire.

En pratique, pour guider chaque vis de fixation, ainsi que, préalablement, un ou plusieurs outils de préparation osseuse, tels qu'un foret de perçage, ces éléments sont introduits et déplacés jusqu'à l'os à l'intérieur d'une tige creuse de guidage, que l'on qualifie également de manchon ou de douille, cette tige de guidage étant reçue de manière ajustée et coulissante dans l'un des trous de l'ancillaire de visée. Toutefois, à défaut d'aménagements ad hoc, cette tige de guidage n'est pas bloquée dans le trou de l'ancillaire, notamment en étant mobile en translation. Le travail du chirurgien n'est alors pas facilité durant l'intervention et manque de précision, en particulier du fait que, sous l'action des chairs entourant l'os, qui sont traversées par l'extrémité distale de la tige de guidage, cette dernière a tendance à être repoussée en direction opposée à l'os.

Pour palier à cet inconvénient, il est connu de FR-A-2 934 145 un ancillaire chirurgical de visée, associé à une lame de blocage de la tige de guidage précitée. Cette lame est agencée de manière à s'étendre en longueur suivant une direction orthoradiale à l'axe du trou de réception de la tige de guidage, la lame étant prévue élastiquement déformable entre une configuration de repos, dans laquelle la tige de guidage reste libre en translation à l'intérieur du trou de l'ancillaire, et une configuration déformée, dans laquelle la lame exerce un effort résilient de blocage, le passage entre les deux configurations précitées étant commandé par une rotation de la tige de guidage sur elle-même autour de l'axe central du trou de l'ancillaire. Bien que cette solution soit mécaniquement satisfaisante en ce qui concerne le blocage de la tige de blocage, elle peut induire une difficulté de mise en oeuvre pour le chirurgien car ce dernier a souvent tendance, avant de chercher à bloquer la tige de guidage, à entraîner cette tige en rotation sur elle-même selon des mouvements angulaires de va-et-vient, notamment afin de faciliter la progression de l'extrémité distale de cette tige de guidage à travers les chairs. Or, si le chirurgien n'y prend pas garde, une telle manipulation rotative de la tige de guidage peut commander des blocages non souhaités de la tige.

Un autre ancillaire à finalité similaire est divulgué dans US-A-2004/059329 sur lequel est basé le préambule de la revendication 1.

Le but de la présente invention est d'améliorer les instrumentations chirurgicales existantes, de manière à ce que ce type d'instrumentation, tout en assurant un blocage efficace d'une tige de guidage telle que celle évoquée ci-dessus, offre une grande liberté opératoire pour le chirurgien.

A cet effet, l'invention a pour objet une instrumentation chirurgicale, telle que définie à la revendication 1.

Une des idées à la base de l'invention est de chercher à coincer la tige de guidage par rapport au trou de l'ancillaire recevant cette tige, en agissant non pas à l'aide d'une lame s'étendant en longueur transversalement à la tige, mais à l'aide d'une pièce dont le corps s'étend en longueur globalement parallèlement à la tige. Pour ce faire, selon l'invention, le corps de cette pièce de coincement présente un relief saillant tourné vers la tige de guidage, tel qu'un bossage, qui est conçu pour s'appuyer transversalement, voire quasi radialement, contre la tige de guidage, lorsque le corps tend, par élasticité, à retrouver sa configuration de repos après avoir été suffisamment déformé pour effacer le relief précité vis-à-vis de la trajectoire d'introduction de la tige de guidage dans le trou de l'ancillaire. Eu égard à l'orientation globale du corps de cette pièce de coincement selon la direction longitudinale du trou de l'ancillaire, on comprend que l'action de blocage, exercée par le relief précité, est commandée à la façon d'un levier par le corps de la pièce, tout en mettant à profit la déformabilité élastique de ce corps pour que cette action de blocage soit induite par retour élastique du corps vers sa configuration de repos. Autrement dit, grâce à l'instrumentation selon l'invention, la tige de guidage est bloquée efficacement en translation par rapport au trou, ce blocage intervenant dès que le chirurgien relâche une contrainte qu'il a préalablement exercée pour déformer élastiquement le corps de la pièce de coincement, nécessaire à l'introduction et au libre coulissement de cette tige dans le trou aux fins de mise en place de cette tige. Ainsi, une fois que la tige de blocage est efficacement bloquée par le relief du corps de la pièce de coincement relâchée par le chirurgien, ce dernier dispose de ses deux mains pour poursuivre une intervention chirurgicale, notamment une intervention visant à mettre en place un clou d'ostéosynthèse, comme expliqué dans la partie introductive de ce document.

Des caractéristiques additionnelles avantageuses de l'instrumentation conforme à l'invention, prises isolément ou suivant toutes les combinaisons techniquement possibles, sont spécifiées aux revendications dépendantes 2 à 10.

L'invention sera mieux comprise à la lecture de la description qui va suivre, donnée uniquement à titre d'exemple et faite en se référant aux dessins sur lesquels :
- la figure 1 est une vue en perspective d'une instrumentation conforme à l'invention, en cours d'utilisation sur un os représenté de manière partielle et schématique ;
- la figure 2 est une vue en perspective d'une pièce de coincement, montrée seule, appartenant à l'instrumentation de la figure 1 ;
- la figure 3 est une coupe partielle dans le plan III de la figure 1;
- les figures 4 et 5 sont des coupes partielles selon les lignes IV-IV et V-V de la figure 3 ;
- les figures 6 et 7 sont des vues similaires à la figure 3, illustrant respectivement deux configurations d'utilisation différentes comparativement à celle illustrée à la figure 3 ; et
- la figure 8 est une vue en perspective d'une variante de réalisation d'une instrumentation conforme à l'invention.

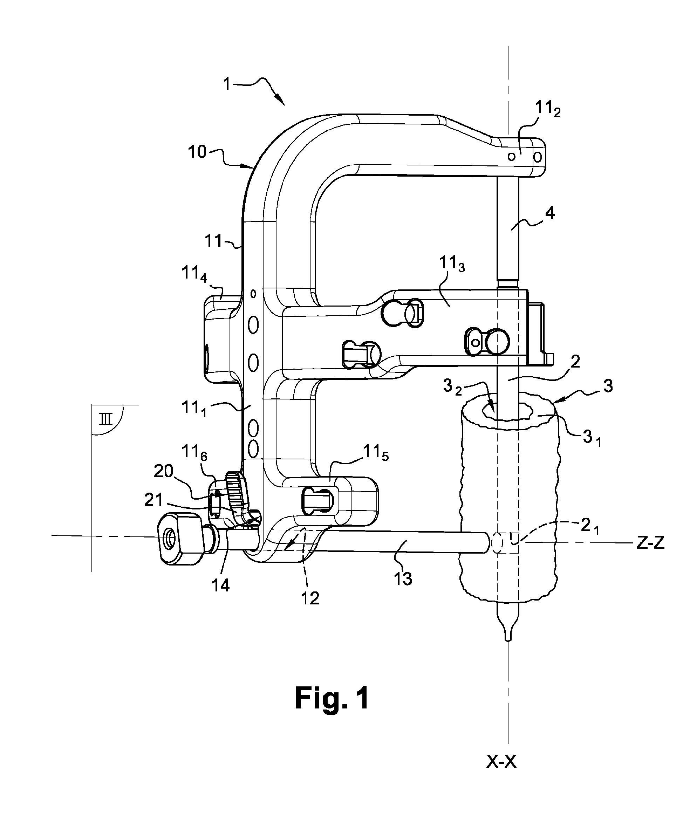

Sur les figures 1 à 7 est représentée une instrumentation chirurgicale 1 conçue pour mettre en place un clou d'osthéosynthèse 2 dans un os long 3. Sur la figure 1, l'os 3 est représenté de manière partielle et schématique, essentiellement pour montrer la forme globalement tubulaire de cet os, avec sa paroi osseuse référencée 3₁ tandis que son volume interne libre, qui correspond au canal médullaire de l'os, est référencé 3₂. A titre d'exemple, l'os long 3 est un humérus, un fémur ou un tibia, de sorte que, dans la position anatomique de ces trois exemples d'os long, le canal médullaire 3₂ est globalement centré sur un axe sensiblement vertical, comme représenté sur la figure 1.

Le clou d'osthéosynthèse 2 considéré ici est un composant connu en soi. Il ne sera donc pas détaillé plus avant, ni représenté en détail, étant simplement remarqué que ce clou présente une forme extérieure globale de nature cylindrique, centrée sur un axe X-X. L'implantation du clou 2 consiste à placer ce clou à l'intérieur de l'os 3, en le faisant s'étendre en longueur à l'intérieur du canal médullaire 3₂, de manière globalement coaxiale, comme montré schématiquement sur la figure 1.

Egalement de manière connue en soi, le clou 2 est traversé, de part en part, par des passages radiaux à l'axe X-X, non représentés à l'exception d'un de ces passages, référencé 2₁. Les passages précités sont répartis suivant la direction longitudinale du clou 2, en étant centrés sur des axes géométriques respectifs qui sont généralement orientés de manière différente dans un plan perpendiculaire à l'axe X-X. Chacun de ces passages, notamment le passage 2₁, est adapté pour recevoir une vis ou un élément de fixation similaire, non représenté, pour fixer transversalement l'un à l'autre le clou 2 et l'os 3.

L'instrumentation 1 comprend un ancillaire de visée 10 conçu pour repérer, de manière extra-cutanée, le positionnement des passages précités du clou 2, notamment le passage 2₁, alors que le clou est logé dans le canal médullaire 3₂ de l'os 3. Pour ce faire, dans l'exemple de réalisation considéré ici, le dispositif de visée 10 comprend un corps 11 dont la partie principale 11₁ présente une forme allongée rectiligne, destinée, en service, à s'étendre de manière globalement parallèle à l'os 3, tout en étant placé à l'extérieur des chairs et autres parties molles entourant cet os. Dans le cas d'exemple d'un clou huméral, cette partie de corps 11₁ s'étend ainsi le long du bras d'un patient, avec interposition des parties molles de ce bras entre la partie 11₁ et l'os de l'humérus.

A l'une de ses extrémités longitudinales, la partie de corps 11₁ est prolongée par une partie de corps 11₂ coudée, qui est dimensionnée, pour, en service, venir coiffer l'extrémité de l'os 3 où débouche le canal médullaire 3₂. A son extrémité libre, cette partie de corps 11₂ supporte une vis 4 de liaison mécanique avec le clou 2. Lorsque le clou 2 est assemblé au corps 11 par cette vis 4, le positionnement angulaire du clou 2 autour de son axe X-X est imposé de manière prédéterminée.

Dans sa région courante, la partie de corps 11₁ est munie de deux parties de corps latérales saillantes 11₃ et 11₄, formant chacune un arceau enveloppant l'os 3 de manière orthoradiale. Ainsi, dans un plan de coupe transversal à la partie de corps 11₁ passant par les parties de corps 11₃ et 11₄, le corps 11 présente globalement un profil en C. De même, dans la région d'extrémité opposée à la partie de corps 11₂, la partie de corps 11₁ est munie de deux parties de corps latérales saillantes 11₅ et 11₆, formant chacune un arceau similaire aux parties de corps 11₃ et 11₄ mais moins étendu que ces dernières de part et d'autre de la partie de corps 11₁.

Comme bien visible sur la figure 1, chacune des parties de corps 11₁, 11₃, 11₄, 11₅ et 11₆ est pourvue de trous traversants, dont celui de la partie de corps 11₁, qui est située à l'extrémité de cette partie de corps, opposée à la partie de corps 11₂, est référencé 12. Ces trous traversants présentent la particularité d'être centrés sur des axes géométriques respectifs qui, lorsque le clou 2 est fixé au corps 11 par la vis de liaison 4, s'étendent radialement par rapport à l'axe X-X du clou 2, en étant respectivement alignés avec l'un des passages précités du clou. En particulier, l'axe Z-Z du trou 12 est aligné avec le passage 2₁. Chacun de ces trous traversants est conçu pour être intérieurement muni d'une tige tubulaire, dont la section transversale est centrée sur l'axe Z-Z et est extérieurement ajustée sur la section transversale intérieure du trou correspondant : sur la figure 1, une seule de ces tiges est représentée, en portant la référence 13 et en étant reçue dans le trou 12. Par la suite, on va décrire en détail la coopération entre le trou 12 et le manchon 13, étant entendu que des considérations similaires s'appliquent à tous les autres trous traversants précités de l'ancillaire de visée 10.

Ainsi, la tige 13 est dimensionnée pour être reçue et guidée de manière coulissante dans le trou 12. En service, le volume délimité intérieurement par la tige 13, qui est ici cylindrique à base circulaire, est utilisé pour guider un outil de préparation percutanée de l'os 3 en vue de mettre en place une des vis de fixation transversale dans le passage 2₁ du clou 2, étant remarqué que cette vis de fixation peut, elle aussi, être guidée intérieurement par la tige 13 : en effet, le volume interne de la tige 13 est centré sur l'axe Z-Z et, grâce au corps 11 de l'ancillaire de visée 10, est aligné de manière coaxiale avec le passage 2₁. Ainsi, en utilisant la tige 13 pour, par exemple, guider un foret de perçage, ce foret agit de manière percutanée pour percer transversalement la paroi 3₁ de l'os 3, depuis la face externe de cette paroi jusque dans le canal médullaire 3₂, la percée osseuse réalisée étant alignée sur le passage 2₁.

En pratique, la forme du corps 11, plus particulièrement de ses parties 11₁, 11₂, 11₃, 11₄, 11₅ et 11₆, n'est pas limitative pour l'instrumentation 1, tant que l'ancillaire de visée 10 permet de positionner des tiges de guidage, telles que la tige 13, de manière extracutanée et par rapport au clou 2, lorsque ce clou est logé dans le canal médullaire 3₂ de l'os 3.

L'instrumentation 1 comprend également un système 20 permettant de bloquer mécaniquement la tige de guidage 13 par rapport au corps 11 de l'ancillaire de visée 10, lorsque cette tige est reçue dans le trou traversant 12. Ce système 20 permet notamment de bloquer en translation la tige de guidage 13 le long de l'axe Z-Z. Bien entendu, l'instrumentation 1 comprend avantageusement, de manière non représentée, plusieurs systèmes, similaires au système 20, respectivement associés aux différentes tiges de guidage présentes et reçues dans autant de trous traversants du corps 11. Ci après, est détaillé plus avant le système 20, étant entendu que des considérations similaires sont valables pour les autres systèmes non représentés.

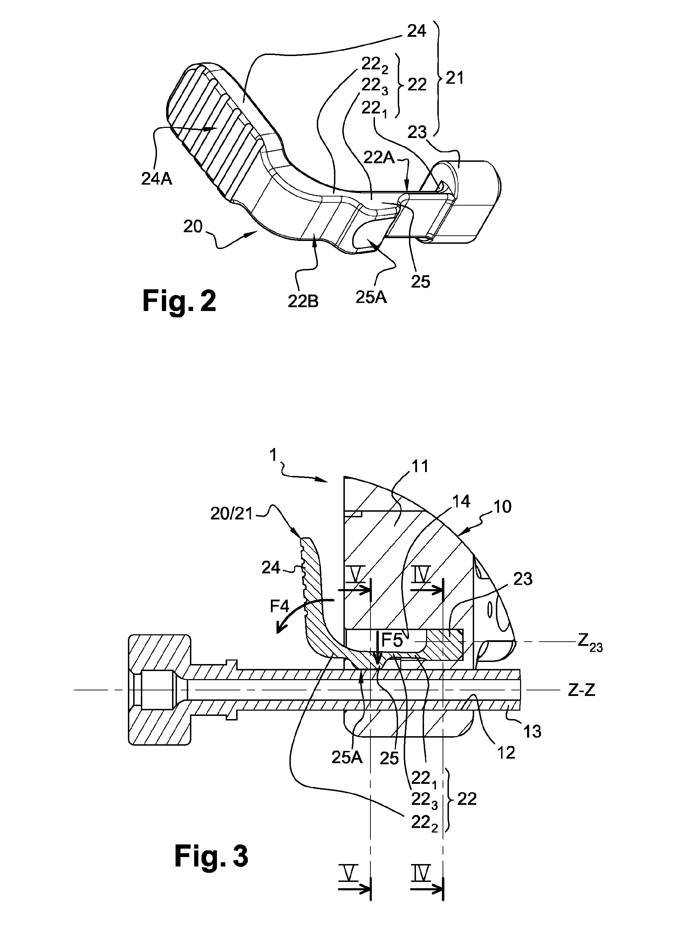

Comme bien visible sur les figures 1 à 3, le système 20 comprend, voire, comme dans l'exemple de réalisation considéré sur les figures 1 à 7, consiste en une pièce spécifique 21, montrée seule à la figure 2. Considérée isolément, cette pièce 21 peut être décrite comme comprenant un corps principal allongé 22, qui présente globalement la forme d'une plaquette et qui s'étend en longueur entre deux extrémités longitudinales opposées 22₁ et 22₂, reliées l'une à l'autre par une partie longitudinale courante 22₃ du corps 22.

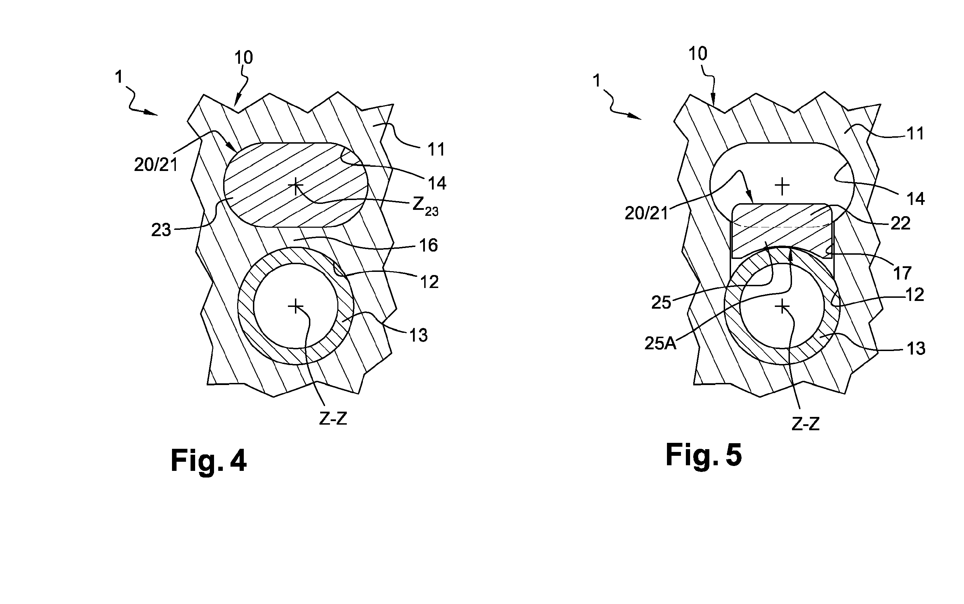

Comme bien visible sur la figure 2, l'extrémité 22₁ est pourvue, de manière saillante et dans le prolongement longitudinale du reste du corps 22, d'un ergot massif 23, qui est fixement solidaire du corps 22, notamment en étant venu de matière avec l'extrémité 22₁, et qui présente, en coupe transversale, c'est-à-dire dans un plan de coupe perpendiculaire à la direction longitudinale du corps 22, une section à profil extérieur oblong, comme bien visible à la figure 4. De plus, comme représenté sur les figures 2 et 3, l'axe longitudinal central Z₂₃ de l'ergot 23, sur lequel est centré le profil oblong précité, ne s'étend pas dans l'épaisseur du corps 22, mais est agencé exclusivement d'un côté de ce corps 22, en s'étendant à l'extérieur de ce corps, à la fois suivant une direction sensiblement parallèle à la direction longitudinale du corps 22 et à distance d'une de ses deux faces principales opposées, référencée 22A.

A l'opposé, l'extrémité 22₂ du corps 22 est, elle aussi, prolongée de manière saillante par une palette 24, qui est fixement solidaire de l'extrémité 22₂, notamment en étant venue de matière avec le corps 22, et qui s'étend à l'opposé du reste du corps 22 de manière coudée, en étant tournée du côté de la face 22A du corps 22, comme bien visible sur les figures 2 et 3.

Par ailleurs, dans sa partie courante 22₃, le corps 22 porte fixement un bossage 25, qui fait saillie de la face principale 22B du corps 22, opposée à la face 22A, et qui émerge du reste de cette face 22B globalement suivant une direction transversale à la direction longitudinale du corps 22. Ce bossage 25 présente ainsi, à l'opposé de la face 22A suivant une direction perpendiculaire au plan global que forme le corps 22, une surface 25A qui s'étend à la fois, et ce dans des proportions respectives sensiblement similaires pour le mode de réalisation considéré sur les figures, selon la direction longitudinale du corps 22 et selon une direction perpendiculaire à cette dernière, reliant les bords transversaux opposés du corps 22. Avantageusement, comme bien visible sur la figure 5, cette surface 25A présente, en coupe perpendiculaire à la direction longitudinale du corps 22, un profil incurvé concave. A titre d'exemple, la surface 25A correspond globalement à une portion de surface cylindrique, centrée sur un axe géométrique s'étendant du côté de la face principale 22B du corps 22, de manière sensiblement parallèle ou inclinée par rapport à la direction longitudinale de ce corps, comme expliqué plus en détail par la suite.

Pour des raisons qui apparaîtront plus loin, la pièce 21 est constituée d'une ou de plusieurs matières choisies pour que le corps 22 présente une capacité de déformation élastique, notamment une capacité de déformation élastique en flexion entre ses extrémités opposées 22₁ et 22₂. Ainsi, la pièce 21 est par exemple réalisée en un métal ou en un alliage métallique souple, tel que le titane ou un alliage à base de titane. Un autre exemple consiste à choisir une matière plastique semi-rigide, telle que le Radel (marque déposée). Dans tous les cas, les matériaux choisis résistent avantageusement à des opérations de stérilisation du type de celles couramment employées dans le domaine des instrumentations chirurgicales réutilisables.

En tenant compte de ce qui précède, la pièce 21 est, en pratique, avantageusement réalisée sous forme monobloc, c'est-à-dire avec son corps 22, y compris son bossage 25, ainsi qu'avec son ergot 23 et sa palette 24 réalisés d'une seule pièce en une ou plusieurs matières ad hoc.

D'autres caractéristiques relatives à la pièce 21 apparaîtront ci-après dans le cadre de la description d'un exemple d'utilisation de cette pièce avec l'ancillaire de visée 10, en lien avec la présence et le réglage en position de la tige de guidage 13.

Ainsi, initialement, la pièce 21 est totalement séparée de l'ancillaire de visée 10 et présente donc une configuration de repos, illustrée à la figure 2. Cette configuration de repos correspond typiquement à la configuration qu'occupe la pièce 21 à l'issue de ses opérations de fabrication, notamment à l'issue de son usinage final ou à l'issue de sa sortie de moule.

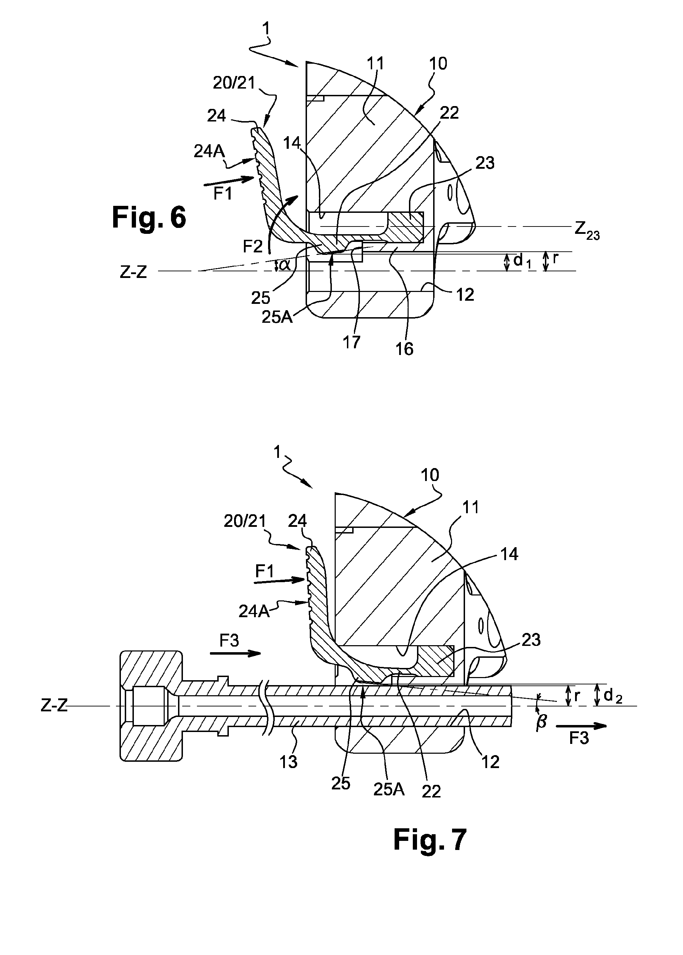

La pièce 21 est alors assemblée à l'ancillaire de visée 10 comme représenté sur la figure 6. Plus précisément, l'ergot 23 est introduit, suivant la direction de son axe Z₂₃, à l'intérieur d'un logement complémentaire 14 délimité par le corps 11 de manière adjacente au trou 12 : comme visible sur les figures 1, 4 et 6, ce logement 14 est agencé parallèlement au trou traversant 12, en étant centré sur un axe parallèle à l'axe Z-Z et en débouchant sur la face proximale du corps 11 qui, en service, est tournée vers le chirurgien, c'est-à-dire, dans le mode de réalisation considéré sur les figures, sur la face de la partie du corps 11₁ opposée à celle tournée vers la partie de corps coudée 11₂. En coupe dans un plan perpendiculaire à l'axe Z-Z, le logement 14 ne présente pas, comme le trou 12, un profil circulaire, mais un profil oblong qui est ajusté sur le profil extérieur de l'ergot 23. Ainsi, en engageant, selon une direction parallèle à l'axe Z-Z, l'extrémité 22₁ du corps 22 dans le logement 14, l'ergot 23 se trouve emmanché de manière ajustée à l'intérieur du logement 14, jusqu'à buter axialement contre le fond de ce logement qui, à la différence du trou 12, est ainsi prévu borgne. La coopération de formes entre l'ergot 23 et le logement 14 immobilise ainsi la pièce 21, à la fois, en rotation sur elle-même autour de l'axe Z₂₃ et en translation selon cet axe en direction opposée au chirurgien. De surcroît, eu égard au frottement entre l'ergot 23 et les parois du logement 14, la pièce 21 est également quelque peu retenue en translation selon l'axe Z₂₃ en direction du chirurgien, tout en gardant la possibilité de la retirer en totalité, en la dégageant du logement sous l'effet d'un effort de traction suffisant en direction du chirurgien : autrement dit, la pièce 21 est liée de manière amovible à l'ancillaire de visée 10.

Dans la configuration de la figure 6, l'essentiel de la partie courante 22₃ du corps 22 est agencé à l'intérieur du logement 14, avec le bossage 25 tourné vers le trou traversant 12. Plus précisément, en raison de la proéminence saillante de ce bossage 25 depuis la face principale 22B du corps 22, la paroi 16 de l'ancillaire de visée 10, séparant le trou traversant 12 et le logement 14 suivant une direction perpendiculaire à l'axe Z-Z, est interrompue du côté proximal du corps 11 de l'ancillaire. Ainsi, comme visible sur les figures 5 et 6, cette paroi 16 est, dans sa partie proximale, fendue de part en part entre le trou 12 et le logement 14, la fente correspondante 17 recevant partiellement le bossage 25, comme représenté sur la figure 5. Avantageusement, l'écartement de cette fente 17 est ajusté sur la dimension transversale du bossage 25, guidant ainsi les mouvements de ce bossage dans une direction perpendiculaire à l'axe Z-Z et à l'axe central du logement 14.

On notera que la coopération de formes entre le bossage 25 et la fente 17 fournit, à l'attention du chirurgien, un détrompage en ce qui concerne l'assemblage de la pièce 21 à l'ancillaire de visée 10, dans le sens où si le chirurgien cherche à engager la pièce 21 avec la face 22A de son corps 22 tournée vers l'axe Z-Z, il sera empêché de mettre en place l'ergot 23 jusque dans le fond du logement 14, par mise en butée axiale du bossage 25 contre la paroi du logement 14 transversalement opposée à la fente 17.

A l'issue de l'assemblage de la pièce 21 à l'ancillaire de visée 10, comme décrit jusqu'ici, l'instrumentation 1 est dans l'état de service représenté à la figure 6, avec la pièce 21 en configuration de repos, portée par l'ancillaire de visée. Comme bien visible sur cette figure 6, le bossage 25 est partiellement agencé à l'intérieur du trou traversant 12 : plus précisément, au moins une partie de la surface 25A, à savoir uniquement la partie proximale de cette surface 25A pour l'exemple considéré sur les figures, se trouve alors située à une distance radiale minimale d₁ de l'axe Z-Z, qui est strictement inférieure au rayon r du profil circulaire du trou traversant 12. On comprend donc que, en l'état, la pièce 21 interdit d'engager à l'intérieur du trou 12 la tige de guidage 13, en raison de l'interférence diamétrale entre ce bossage 25 et la face extérieure de cette tige.

Dans une étape subséquente, visant justement à permettre l'engagement de la tige de guidage 13 dans le trou traversant 12, le chirurgien entraîne en déformation élastique le corps 22 de manière à effacer le bossage 25 de la trajectoire d'insertion de la tige 13. Pour ce faire, le chirurgien appuie sur la palette 24, comme indiqué par la flèche F1 à la figure 6, typiquement à l'aide d'un de ses doigts, notamment à l'aide du pouce de sa main tandis que les autres doigts de cette dernière agrippent la partie de corps 11₁, de manière à éloigner de lui la palette 24, en cherchant à la plaquer contre la face proximale du corps 11, jusqu'à atteindre la configuration de la figure 7. Avantageusement, la palette 24 présente à cet effet une surface proximale 24A nervurée, contre laquelle le pouce ou tout autre doigt du chirurgien frotte pour favoriser la transmission de mouvement. Eu égard à la capacité de déformation élastique du corps 22 et dans la mesure où l'extrémité 22₁ de ce corps est liée de manière sensiblement fixe au corps 11 de l'ancillaire de visée 10, par la coopération de formes entre l'ergot 23 et le logement 14, cet entraînement de la palette 24 et donc de l'extrémité 22₂ du corps 22 induit une déformation globale du corps 22, essentiellement en basculement autour d'un axe géométrique, qui s'étend de manière sensiblement orthoradiale à l'axe Z-Z et qui passe sensiblement par la région de l'extrémité 22₁ du corps 22. Ce mouvement de basculement d'ensemble est indiqué par la flèche F2 sur la figure 6. Plus globalement, le corps 22 est alors soumis à une flexion répartie sur sensiblement toute sa longueur, par décalage transversal de son extrémité 22₂ par rapport à son extrémité 22₁ sensiblement fixe par rapport à l'ancillaire 10, l'extrémité 22₂ étant décalé transversalement à et à l'opposé de l'axe Z-Z.

En raison du fléchissement de la partie courante 22₃ du corps 22 en direction opposée à l'axe Z-Z, induit par l'appui digital sur la palette 24, le bossage 25 est éloigné transversalement vers l'extérieur du trou traversant 12, jusqu'à ce que la distance radiale minimale d₂ séparant sa surface 25A de l'axe Z-Z soit strictement supérieure au rayon r du trou 12, comme sur la figure 7. Dans l'exemple de réalisation considéré, cette distance radiale minimale d₂ est définie entre l'axe Z-Z et la partie distale de la surface 25A. On comprend que la tige de guidage 13 peut alors être introduite dans le trou 12, sans buter contre le bossage 25 ainsi effacé, comme indiqué par les flèches F3 sur la figure 7.

Comme expliqué plus haut, la tige 13 est réglée en position par rapport à l'ancillaire 10, moyennant sa translation le long de l'axe Z-Z, jusqu'à ce que son extrémité distale occupe, vis-à-vis du clou 2 et de l'os 3, la position voulue par le chirurgien, notamment que cette extrémité distale soit au contact de la paroi 3₁ de l'os 3, après avoir traversé les chairs et autres parties molles entourant cette paroi 3₁.

Une fois que le chirurgien a fini de régler la position de la tige de guidage 13 le long de l'axe Z-Z par rapport à l'ancillaire de visée 10, le chirurgien relâche l'appui digital qu'il avait maintenu jusqu'alors sur la palette 24. Comme indiqué par la flèche F4 sur la figure 3, la pièce 21 cherche alors, par retour élastique, à reprendre sa configuration de repos de la figure 6. Cependant, eu égard à la présence de la tige de guidage 13 à l'intérieur du trou traversant 12, le bossage 25 est empêché de retrouver sa position de la figure 6, sa surface 25A s'appuyant alors, transversalement à l'axe Z-Z, voire sensiblement radialement à cet axe, contre la face extérieure de la tige de guidage 13, comme indiqué par la flèche F5 sur la figure 3. On comprend que le corps 22 occupe alors une position intermédiaire entre sa configuration déformée de déblocage, illustrée à la figure 7, et sa configuration de repos illustrée à la figure 6, étant remarqué que cette configuration intermédiaire de la figure 3 correspond à une configuration déformée pour le corps 22, qui peut être qualifiée de configuration déformée de blocage vis-à-vis de la tige 13. En effet, par appui transversal, voire radial de la surface 25A contre la face extérieure de la tige 13, cette dernière se trouve coincée dans le trou traversant 12, dans le sens où, par frottement mécanique direct entre la surface 25A et la face extérieure de la tige 13, cette dernière est empêchée d'être déplacée suivant l'axe Z-Z.

On notera que deux aménagements avantageux renforcent significativement la coopération de frottement entre la surface 25A et la tige de guidage 13. Un premier aménagement est relatif au profil concave de la surface 25A, comme expliqué plus haut en regard de la figure 5. Grâce à ce profil concave, l'interface de frottement entre le bossage 25 et la tige de guidage 13 présente une grande étendue de part et d'autre d'un plan contenant les axes Z-Z et Z₂₃.

Selon le second aménagement, que présente le mode de réalisation représenté sur les figures 1 à 6, le bossage 25 est conçu pour que, lorsque le corps 22 est dans la configuration déformée de blocage de la figure 3, sa surface 25A présente, dans un plan de coupe longitudinale du corps 22 parallèle à l'axe Z-Z, comme dans le plan de coupe de la figure 3, un profil rectiligne parallèle à l'axe Z-Z : de la sorte, dans le plan de coupe longitudinale précité, l'interface de frottement entre le bossage 25 et la tige de guidage 13 est étendue, en mettant à profit toute la dimension longitudinale de la surface 25A. En pratique, eu égard à la déformation en flexion que suit le corps 22 lors de son passage successif entre sa configuration de repos, sa configuration déformée de déblocage et sa configuration déformée de blocage, cela se traduit par le fait que le profil rectiligne précité de la surface 25A passe d'une inclinaison initiale, qui converge vers l'axe X-X en direction de l'extrémité 22₂ du corps 22, en formant avec l'axe Z-Z un angle α, lorsque le corps 22 est dans sa configuration de repos, à une inclinaison de sens opposé, convergente vers l'axe X-X en direction de l'extrémité 22₁, en formant avec l'axe Z-Z un angle β sensiblement opposé à l'angle α lorsque le corps 22 est dans sa configuration déformée de déblocage, comme bien visible par comparaison des figures 6 et 7.

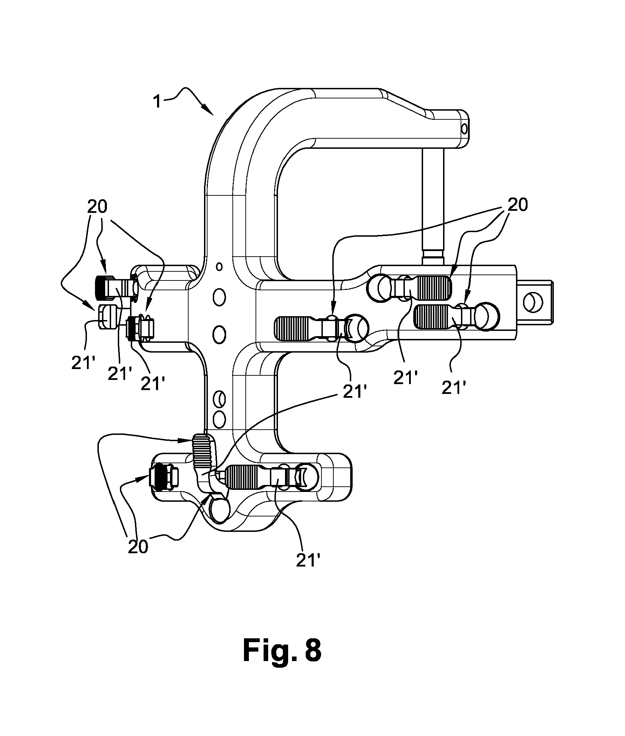

Sur la figure 8 est représentée une variante de réalisation de l'instrumentation 1 décrite jusqu'ici en regard des figures 1 à 7. Cette variante de l'instrumentation 1 se distingue exclusivement par ses systèmes de blocage 20 : à la différence de la pièce 21 des figures 1 à 7, la pièce 21' de chaque système 20 de la figure 8 est solidarisée à demeure à l'ancillaire de visée 10, ce qui explique pourquoi cet ancillaire est représenté à la figure 8 avec tous ses logements 14 recevant l'une des pièces 21' précitées. En pratique, chacune de ces pièces 21' présente une structure individuelle identique à celle de la pièce 21, la différence entre chaque pièce 21' et la pièce 21 venant du fait que, dans le fond de chacun des logements 14 recevant les pièces 21', un élément de solidarisation à demeure est prévu, tel qu'un point de colle ou une couche d'adhérent chimique qui se trouve interposé entre l'ergot, non visible sur la figure 8, de la pièce 21' et la paroi du logement 14 correspondant. Bien entendu, d'autres possibilités peuvent être envisagées pour lier à demeure chacune des pièces 21' à l'ancillaire de visée 10, tout en permettant à ces pièces 21' de pouvoir être déformées élastiquement entre une configuration de repos, similaire à celle montrée à la figure 6 pour la pièce 21, et une configuration déformée de déblocage, similaire à celle montrée sur la figure 7 sur la pièce 21, en passant par une configuration intermédiaire de blocage, telle que celle montrée à la figure 3 pour la pièce 21.

Divers aménagements et variantes à l'instrumentation décrite jusqu'ici sont par ailleurs envisageables. A titre d'exemple plutôt que d'être à profil transversal incurvé concave, la surface 25A du bossage 25 peut être prévue sensiblement plate, en étant notamment agencée de manière inclinée sous l'angle α par rapport à l'axe Z-Z lorsque le corps 22 est dans sa configuration de repos et que la pièce 21 est assemblée à l'ancillaire de visée 10.

## Revendications

1. Instrumentation chirurgicale (1), comprenant :
- un ancillaire (10), notamment un ancillaire de visée pour implanter un clou d'osthéosynthèse (2), lequel ancillaire délimite au moins un trou traversant (2) sensiblement cylindrique, définissant un axe central (Z-Z) et étant adapté pour recevoir co-axialement une tige (13) qui présente une section transversale ajustée à celle du trou traversant, et
- des moyens de blocage (20) qui sont portés par l'ancillaire (10), qui sont adaptés pour bloquer en translation axiale la tige (13) par rapport au trou traversant (12) et qui comprennent une pièce (21 ; 21') de coincement de la tige (13) dans le trou traversant (12), cette pièce de coincement comprenant un corps allongé (22) qui est élastiquement déformable entre une configuration de repos et une configuration déformée,
**caractérisée en ce que** le corps (22) en service s'étend en longueur de manière globalement parallèle à l'axe central (Z-Z),
**en ce que**, dans les configuration de repos et déformée du corps (22), un relief (25), qui fait transversalement saillie du corps en direction de l'axe central, est écarté vis-à-vis de l'axe central respectivement d'une distance radiale minimale (d₁) strictement inférieure au rayon du trou traversant (12) et d'une distance radiale minimale (d₂) strictement supérieure au rayon du trou traversant,
et **en ce que**, entre ses configurations de repos et déformée, le corps (22) passe par une configuration intermédiaire dans laquelle, alors que la tige (13) est reçue dans le trou traversant, une surface d'appui (25A) délimitée par le relief (25) est pressée contre la tige selon une direction transversale à l'axe central (Z-Z) sous l'action du retour élastique du corps (22) depuis sa configuration déformée vers sa configuration de repos.

2. Instrumentation suivant la revendication 1, **caractérisée en ce que**, en coupe perpendiculaire à l'axe central (Z-Z), la surface d'appui (25A) du relief (25) présente un profil incurvé concave.

3. Instrumentation suivant l'une des revendications 1 ou 2, **caractérisée en ce que**, dans un plan de coupe longitudinal du corps (22), parallèle à l'axe central (Z-Z), la surface d'appui (25A) du relief (25) présente un profil sensiblement rectiligne qui, lorsque le corps est passé de sa configuration de repos à sa configuration déformée, passe d'une inclinaison (α) dans un sens à une inclinaison (β) en sens opposé par rapport à l'axe central (Z-Z), ce profil sensiblement rectiligne étant avantageusement parallèle à l'axe central lorsque, en présence de la tige (13), le corps est dans sa configuration intermédiaire.

4. Instrumentation suivant l'une des revendications précédentes, **caractérisée en ce que** la pièce de coincement (21 ; 21') comprend, à une première (22₁) des extrémités longitudinales (22₁, 22₂) de son corps (22), un moyen (23) de liaison sensiblement fixe avec l'ancillaire (10).

5. Instrumentation suivant la revendication 4, **caractérisée en ce que** le moyen de liaison inclut, voire consiste en un ergot (23), solidaire de la première extrémité (22₁) du corps (22) et adapté pour coopérer par complémentarité de formes avec un logement dédié (14) délimité par l'ancillaire (10).

6. Instrumentation suivant l'une des revendications 4 ou 5, **caractérisée en ce que** le moyen de liaison (23) est conçu pour lier la pièce de coincement (21) à l'ancillaire (10) de façon amovible.

7. Instrumentation suivant l'une des revendications 4 ou 5, **caractérisée en ce que** le moyen de liaison inclut un élément de solidarisation à demeure de la pièce de coincement (21') à l'ancillaire (10), tel qu'un élément de collage ou un élément d'adhésion chimique.

8. Instrumentation suivant l'une quelconque des revendications précédentes, **caractérisée en ce que** la pièce de coincement (21 ; 21') comprend, à une seconde (22₂) des extrémités longitudinales (22₁, 22₂) de son corps (22), un moyen (24) d'entraînement du corps en déformation depuis sa position de repos à sa position déformée, adapté pour entraîner le corps en flexion longitudinale, notamment globalement en basculement autour d'un axe géométrique orthoradial à l'axe central (Z-Z).

9. Instrumentation suivant la revendication 8, **caractérisée en ce que** le moyen d'entraînement inclut, voire consiste en une palette d'appui digital (24), solidaire de la seconde extrémité (22₂) du corps (22).

10. Instrumentation suivant la revendication 9, **caractérisée en ce que** la palette d'appui digital (24) est coudée par rapport au corps (22) et présente une surface proximale (24A) nervurée.
